(19) **Europäisches Patentamt / European Patent Office / Office européen des brevets**

(11) **EP 1 787 579 A1**

(12) **EUROPEAN PATENT APPLICATION**
published in accordance with Art. 158(3) EPC

(43) Date of publication:
**23.05.2007 Bulletin 2007/21**

(21) Application number: **05774468.2**

(22) Date of filing: **26.07.2005**

(51) Int Cl.:
**A61B 3/02** *(2006.01)*    **A61B 3/06** *(2006.01)*

(86) International application number:
**PCT/ES2005/070109**

(87) International publication number:
**WO 2006/024687 (09.03.2006 Gazette 2006/10)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.07.2004 ES 200401961**

(71) Applicants:
• **Universitat de Valencia**
  **46100 BURJASSOT (ES)**
• **INDO Internacional, S.A.**
  **08174 Sant Cugat del Valles (ES)**

(72) Inventors:
• **ARTIGAS VERDE, José María**
  **Dpto. de Óptica**
  **E-46100 BURJASSOT (ES)**

• **CAPILLA PEREA, Pascual**
  **Dpto. de Óptica**
  **E-46100 BURJASSOT (ES)**
• **LUQUE COBIJA, María José**
  **Dpto. de Óptica**
  **E-46100 BURJASSOT (ES)**
• **ALBERT SESEÑA, Santiago**
  **E-08902 L'HOSPITALET DE LLOBREGAT (ES)**
• **MÁRQUEZ PÉREZ, Véronica**
  **E-08902 L'HOSPITALET DE LLOBREGAT (ES)**
• **FELIPE MARCET, Adelina**
  **Dpto. de Óptica**
  **E-46100 BURJASSOT (ES)**

(74) Representative: **Barlocci, Anna**
  **Zea, Barlocci & Markvardsen, S.L.**
  **Balmes, 114 4o**
  **08008 Barcelona (ES)**

(54) **DEVICE AND METHOD FOR DETERMINING VISION CONTRAST SENSITIVITY OF AN INDIVIDUAL**

(57)    Device to determine contrast sensitivity of an individual's eye, comprising means for producing real time digital images corresponding to modulated visual stimuli and display means (D) for presenting these images to the eye, and corresponding method using said device. The device performs a series of tests in a measurement session, so that the stimuli of said tests are modulated around an average stimulus in any direction of the color space and they can be configured for each measurement session. Said stimuli are usually chosen in the cardinal directions of the color space and respond to a sinusoidal grid pattern with spatial and temporal modulation, and with two dimensional gaussian envelope.

FIG. 1

EP 1 787 579 A1

**Description**

The present invention relates to a device to determine the contrast sensitivity of an individual's visual system by a series of tests during a measurement session, comprising means for producing real time digital images, corresponding to modulated visual stimuli, and display means for presenting these images to an individual's eye, and a corresponding method using said device.

**Background of the invention**

Visual information travels from the retina to primary visual cortex or striatum following two parallel ways referred to as magnocellular and parvocellular ways. These ways have their origin in the ganglionar cells called M and P, respectively. 75% of magnocellular way cells (referred to as MX) is linearly responsible whereas 25% of said cells (referred to as My) is nonlinear. All the parvocellular cells are linearly responsible.

There are many evidences proving that the detection of a purely chromatic contrast pattern, whether it be red-green or blue-yellow, is mediated by cells with spectral opponency belonging to parvocellular way. Therefore, this way would be revealed as the physiological support for detection of chromatic contrasts in the whole space-time domain. Detection of an achromatic contrast pattern is much more controversial, although some studies point that the magnocellular way is the physiological support for detection of chromatic contrasts in the corner of low spatial frequencies and high temporal frequencies within space-time domain.

A large variety of pathologies result in important injuries in one of these ways, and possibly in both. The origin of the pathology can be found in the retina, in the optic nerve, chiasma or any other site in the visual ways and even in visual cortex. Whatever is the extent and the magnitude of the damage caused, it has been proven that contrast sensitivity on the damaged way will be altered. In any case, it is important to know which pathologies impair the Magno, which the Parvo and which both, and in each case, which is the most suitable strategy for detecting a reduction in sensitivity on the damaged way or ways.

From the above it follows that when only the Magno is damaged by a pathology, a reduction of sensitivity in the visual system should be revealed by using an achromatic pattern of low spatial frequencies and high temporal frequencies. Nevertheless, an injury in the Parvo will result in a reduction of sensitivity in an wide set of spatial-temporal conditions with achromatic patterns and in any spatial-temporal conditions with modulated isoluminant patterns in any directions of the space color, specifically, the cardinal directions of the red-green or blue-yellow mechanisms. It has been not sufficiently proven which of the predictable reductions is previous in the progress of the pathology nor which is the one of highest magnitude. It has also been not sufficiently proven what deterioration is previous and more significant when both Magno and Parvo visual ways are injured by pathology. On the other hand, it is well known that the possibilities of making a good patient's diagnosis are significantly enhanced if a scanning of the whole retina and not only of the fovea is performed, that is, if what in the clinical language is referred to as campimetry is performed. In its more traditional form, a campimetry consists in measuring, at each position of the field of sight, the threshold luminance so that a simple white circular spot can be detected on a background with a certain luminance, also white. However, in this type of campimetry sensitivity losses are detected when a significant important amount of ganglionar cells, for example in glaucoma, has been already damaged.

In recent years, notable efforts have been made to improve expectations in campimetric scanning. In the technique referred to as SWAP (Short Wavelength Automated Perimetry) threshold is determined for detecting a blue circular spot on a yellow background. It has been shown that the detection of a spectral stimulus of a certain wavelength on a white background is mediated by ganglionar cells that are more sensitive for that wavelength. Blue spot campimetry improves the results which would be obtained traditionally with white on white campimetry. But a yellow background will increase the probability that the blue stimulus is detected by the blue-yellow channel. Although SWAP has substantially improved the features of the campimetries on white background, is not still a technique free of problems. For example, it is well known that the response of a chromatic or achromatic mechanism is not isolated by an incremental paradigm (that is, a stimulus represented on a background), since it simultaneously entails a variation in the luminance and a variation in the color.

On the other hand, it has been proposed that the detection of a sinusoidal achromatic pattern in space and time with suitable frequencies is capable of isolating the response of magnocellular way cells. These cells are affected, like the cells with blue-yellow opponency, by different pathologies, including glaucoma. If spatial frequency is particularly low and temporal frequency is particularly high the pattern is perceived as if spatial frequency was twice as much as it actually is. It has been suggested that this phenomenon, referred to as frequency doubling, is attributable to *My* cells nonlinear behavior. The technique related to frequency doubling observation is referred to as FDT (Frequency Doubling Technology).

US patent US5065767 discloses a method according to which a patient is presented a sinusoidal grid pattern which contrast is modulated to a frequency ranging from 10 to 50 Hz. Patient initially observes a spatial frequency doubling in this grid. Contrast resistance is gradually being reduced until a threshold value is reached with which the patient stops observing the frequency doubling. In patients with glaucoma this threshold can be as much as twice the contrast an observer considered as normal would need.

On the other hand, US patents US6068377 and US6227668 provide methods extending the above idea, in the sense that the frequency doubling would also occur with any pattern which color palette consists of mixtures of complementary colors, particularly, blue and yellow mixtures. The pattern must be periodic in space and time and, as in the achromatic frequency doubling, spatial frequency must be low (non greater than 5 cycles/degree) and temporal frequency must be high (non smaller than 7 cycles/seconds). *My* cells are probably also responsible for this phenomenon, so a stimulus capable of producing a chromatic frequency doubling would a priori gather a number of potentially useful conditions for evaluating of problems that simultaneously involve deteriorations in Magno cells and Parvo cells with blue-yellow opponency. Nevertheless, it has not been proven at the moment that this type of campimetry is more effective than campimetry based upon achromatic frequency doubling.

Also with regard to frequency doubling, PCT application WO 95/29627 discloses a method that includes measuring, for different areas of a patient's field of sight, the minimum contrast with which this patient can still perceive an achromatic frequency doubling. This method intends to early detect damages caused in the retina by glaucoma that affects only one part of the patient's field of sight.

In any case, it seems clear that a combination of two more tests can improve the precocious diagnosis of ocular pathologies, since some tests can detect before others the same sight defects but caused by different pathologies. For example, sometimes an optic neuropathy can be identified earlier by means of stereophotographies than by detecting a sensitivity loss; or, on the contrary, a sensitivity loss is often detected in glaucoma first by SWAP or FDT tests. In any case, both SWAP and FDT, with some advantage for FDT, is revealed to be quite effective as precocious indicators of sensitivity loss in glaucoma.

**Description of the invention**

An object of the invention is obtaining a high performance device which makes possible providing a very wide range of measurements and tests of the type being proposed so far, including chromatic and achromatic frequency doubling, and which also allows to explore other questions still open to discussion, such as for example studying if a blue-yellow stimulus producing frequency doubling is more effective for detecting glaucoma or a particular pathology that a pattern with blue-yellow modulation in any other area of the spatial-temporal domain, or if a stimulus with the spatial frequency and the temporal frequency in the area of maximum sensitivity first highlights the presence of a problem, or if achromatic patterns in the area of maximum sensitivity are equal or more effective for detecting a problem in the parvocellular way than even any pattern with blue-yellow modulation, or if certain pathologies entailing injuries in cells with opponency red-green can be detected with greater effectiveness by stimuli with this class of modulation, etc.

According to one aspect of the invention, contrast sensitivity of the visual system is determined by means of a device that makes a series of tests in a measurement session, such that the stimuli of these tests are modulated around an average stimulus in any direction of the color space and can be configured in their spatial and temporal characteristics for each measurement session. Said sensitivity can be determined in the whole spatial-temporal domain and in any direction of the color space, that is a form of representation of the responses of the mechanisms A, T and D, where A is a non opponent or achromatic mechanism, and T and D are mechanisms with red-green and blue-yellow opponency, respectively.

These stimuli are usually chosen in the directions of the color space in which only one of the mechanisms A, T or D is able to respond to the stimulus, which are so called cardinal directions of the color space.

By default, this average stimulus corresponds to the white color of the displaying means.

Advantageously, stimuli are sinusoidally time-modulated.

In one embodiment, these stimuli correspond to a sinusoidal grid pattern with spatial and temporal modulation.

In one embodiment, said sinusoidal grid is modulated, in turn, through a two dimensional gaussian envelope.

The spatial frequency of this pattern is usually selected from 0 to 20 cycles/degree and its spatial modulation takes the horizontal direction. The temporal frequency of the pattern is selected from 0 to 30 cycles/second. Length of each stimulus is selected from 0 to 5 seconds.

In one embodiment, the colors included in the modulated stimuli in a particular direction of the color space are extracted from a 256 color palette generated for said direction.

One embodiment comprises a camera for recording the eye position during a test of the measurement session.

One embodiment comprises means for the individual to hold his/her head and gaze as steady as possible in each test of the measurement session. Said means comprise a chin rest with occluder and support for lenses.

In one embodiment, the individual's field of sight is examined up to 20° in vertical and 30° in horizontal around a fixation point and the fixation point is allowed to be moved up to 20° in vertical and 30° in horizontal. In this way, by varying the position of the fixation point it is possible to examine the individual's field of sight up to 40° in vertical and

60° in horizontal.

In order to examine the individual's field of sight, one embodiment divides it into in sectors, establishing a specific sector for the fovea.

In one embodiment said means for producing images and said displaying means comprise a computing system which further comprises a central processing unit and a memory.

Advantageously said memory comprises: a measurement session configuration and control routine; a measurement session results repository; a repository of results obtained for individuals with no visual pathologies, referred to as standard observers; a visual pathology diagnosis routine from the results of the measurement session and said standard observers; a displaying routine of said results and said diagnosis.

In one embodiment, said routine of measurement session configuration and control comprises: a stimuli configuration subroutine; a measurement method configuration subroutine; a spatial and temporal randomization subroutine of stimuli representation; a stimuli amplitude selection subroutine; a real time recording subroutine of the results of the measurement session.

One embodiment comprises means for indicating detection, if this is the case, on the part of the individual about a spatial-temporal change with respect to average stimulus.

According to another aspect of the invention, contrast sensitivity is determined by a method comprising the use of a device as the one described in the foregoing paragraphs of this section.

Advantageously, during the measurement session the individual is able to indicate detection, if this is the case, about a spatial-temporal change with respect to the average stimulus.

In one embodiment, in order a detection is counted the individual has to indicate it after a period of time from the appearance of the stimulus but before its disappearance.

Advantageously, during the measurement session the position in which each test stimulus is presented to the eye is randomly varied and the pause between one stimulus and the following one is also randomly varied.

One embodiment comprises a step of determining the individual's blind spot position and size. In this step a high luminance, substantially point shaped, short visual stimulus is presented to the individual's eye successively moving in horizontal and vertical directions, with the individual being able to press a push button when the stimulus is visible to him/her, so that the blind spot position and size are calculated from the area in which the individual does not respond to the stimulus.

The measurement session usually comprises the steps of: presenting a stimulus with the maximum possible value of the amplitude; repeating stimulus presentation by dividing the amplitude successively by 2 as long as a stimulus is detected by the individual, that is, until reversal occurs; repeating stimulus presentation multiplying successively by $2^{1/2}$ the amplitude from non detection until the stimulus has been detected again by the individual, that is, until a new reversal occurs; carrying out both preceding steps but applying a division or multiplication factor of $_2 2^{-n}$, respectively, with n being the reversal number; finishing the session after 4 reversals or 20 presentations; allocating the last amplitude value detected to the threshold; and determining contrast sensitivity as the inverse of said threshold.

In one embodiment, the measurement session further comprises the semi-random presentation of control stimuli of false positives, false negatives and fixation loss.

Advantageously, the method comprises the use of an algorithm for selecting the parameters of each test based upon the results of measurement session previous tests.

**Brief description of the drawings**

For a better understanding of what it has been set out drawings are herein enclosed in which, diagrammatically and only by way of non limiting example, a practical case of embodiment is shown.

In the drawings:

Figure 1 is a diagrammatic depiction of the device;
Figure 2 are images corresponding to visual stimuli;
Figure 3 is a depiction of color space cardinal directions; and
Figure 4 is a graph showing the sensitivity threshold searching method.

**Description of preferred embodiments**

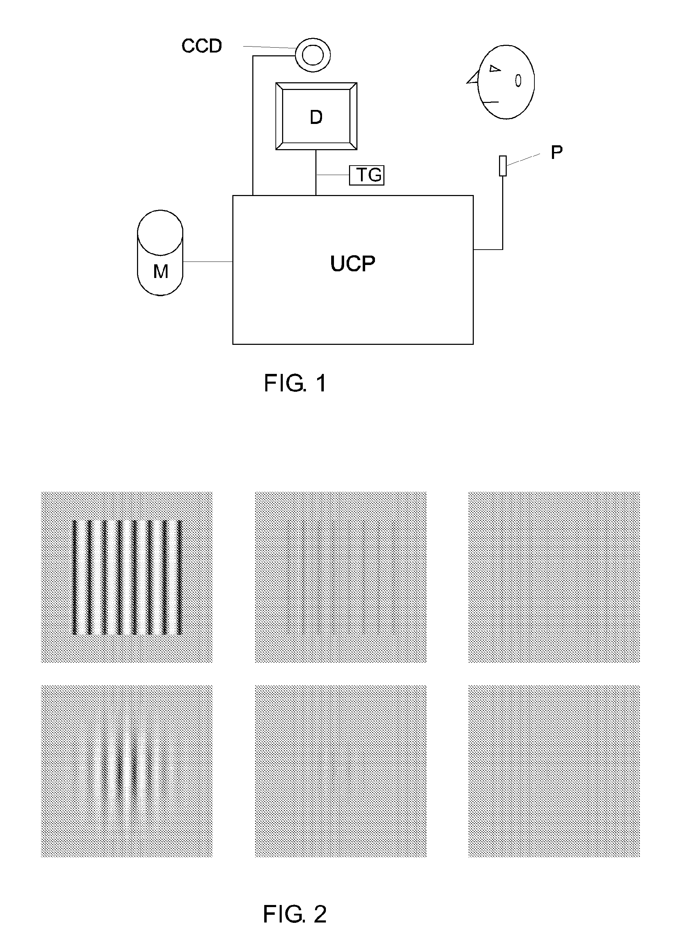

As it can be seen from figure 1, the device of this embodiment comprises a central processing unit UCP, a memory M, a graphics card of at least 14 bits TG, a 17 to 21 inch screen D, a CCD camera for automatic control of fixation, a chin rest with support for lenses and occluder, a monitor for displaying results, a keyboard, a push button P and a color printer. All these elements are built in a single body.

In more detail, said device includes a computer system comprising:

- A central processing unit UCP.
- Means including a graphics card TG for producing real time digital images corresponding to modulated visual stimuli.
- Displaying means D for presenting said images to an individual's eye.
- Means P for indicating detection, if this is the case, on the part of the individual of variations in said stimuli.
- A memory M or hardware for instructions and data comprising:

  1. A measurement session configuration and control routine; this routine in turn comprising:

     - A stimuli configuration subroutine
     - A measurement method configuration subroutine
     - An individual's blind spot position and size determination subroutine
     - A spatial and temporal randomization of individual stimuli displaying subroutine
     - A stimuli amplitude selection subroutine
     - A measurement session results real time recording subroutine.

  2. Repository or data base of measurement session results.
  3. Repository of results obtained for individuals with no visual pathologies, referred to as standard observers.
  4. A diagnosis routine of a visual pathology from measurement session results and standard observers.
  5. A routine for displaying said measurement session and diagnosis results.

The device can be operated in two ways: one with a default menu and another having a menu for performing a customized configuration of the characteristics of the stimulus and the trial parameters.

Features of the device configuration and operation are described below in different sections.

<u>About stimuli chromatic characterization</u>

A stimulus modulated around an average can be described by means of the amplitude $\Delta A$, $\Delta T$, $\Delta D$ from the responses in the mechanisms A, T and D, respectively, where A is a non opponent or achromatic mechanism and T and D are mechanisms with red-green and blue-yellow opponency, respectively. This form of representation is referred to as space color or opponent modulation space. It is applied according to an equation as follows:

$$
\begin{pmatrix} \Delta A \\ \Delta T \\ \Delta D \end{pmatrix} = \begin{pmatrix} K_A & & \\ & K_{\mathbf{T}} & \\ & & K_D \end{pmatrix} \begin{pmatrix} L_0 & M_0 & 0 \\ L_0 & -L_0 & 0 \\ -L_0 & -M_0 & L_0 + M_0 \end{pmatrix} \begin{pmatrix} \dfrac{\Delta L}{L_0} \\ \dfrac{\Delta M}{M_0} \\ \dfrac{\Delta S}{S_0} \end{pmatrix}
$$

where $K_A$, $K_T$, $K_D$ are constants that define the units of measurement in each mechanism.

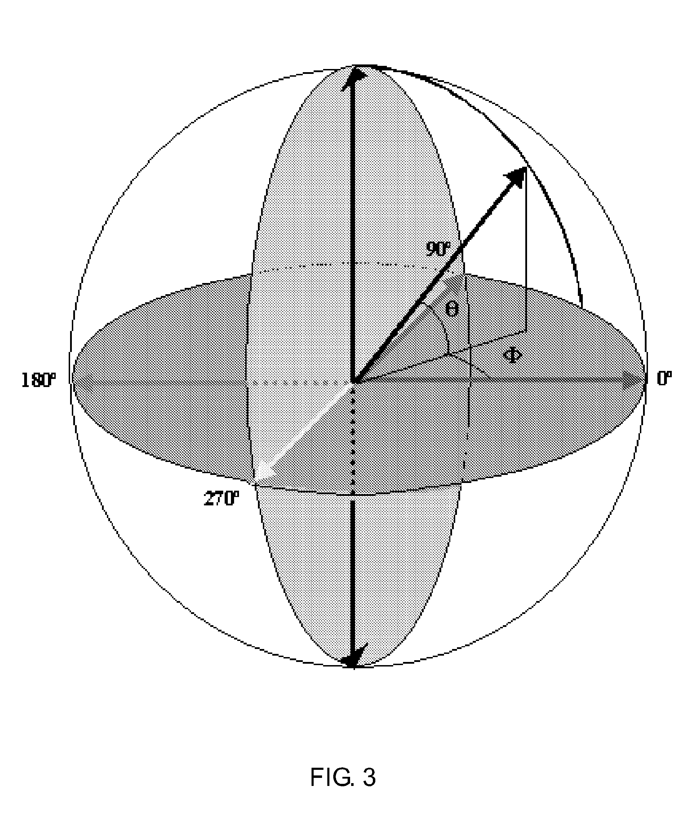

The device allows contrast sensitivity to be evaluated with stimuli modulated around an average stimulus in any direction of the color space. Default generated modulation directions are those in which only one of the mechanisms responds. These directions are referred to as cardinal directions of the color space and are depicted in figure 3.

<u>About the range of colors that can be reproduced by the screen and the way palettes are generated</u>

Maximum stimuli amplitude values isolating each mechanism and which can be reproduced by a particular screen must be explicitly measured for each screen. When the user selects the cardinal direction in which he/she is going to work, a 256 equidistant color palette is automatically generated by a program designed for this purpose in the isolated mechanism response, this being within the range of the maximum and minimum values allowed by the display screen. The number of colors in the palette that will be used for generating a particular image will depend upon the spatial frequency pattern and the image size.

About definition of sensitivity

Contrast absolute threshold is to be measured for detecting a particular sinusoidally modulated stimulus in a cardinal direction of the color space. With luminance or achromatic grids, the so-called Michelson contrast is traditionally used as a metric, which is defined as follows:

$$C = \frac{Y_{MAX} - Y_{MIN}}{Y_{MAX} + Y_{MIN}} = \frac{\Delta Y}{Y_0}$$

where $Y_{MÁX}$ and $Y_{MÍN}$ are the maximum and minimum luminances of the sinusoid, $\Delta Y$ is the amplitude and $Y_0$ is the average luminance. Contrast sensitivity is then defined as follows:

$$CSF(fx, ft) = \frac{1}{C_{UMB}(fx, ft)}$$

This metric can be maintained for grids isolating the achromatic channel, only by substituting amplitude, $\Delta Y$, and average luminance, $Y_0$, for $\Delta A$ and $A_0$, respectively. However, it cannot be applied in the chromatic channels since average values $T_0$ and $D_0$ are approximately zero; they would be in fact strictly zeros if modulation would be done around an equienergetic white. Alternatively, threshold can be estimated as the minimum value of the amplitude for which there is detection. Since the average around which it is modulated is the same for all the measures, amplitude thresholds are proportional to contrast thresholds. Therefore, a *pseudo CSF* in channel A can be defined as follows:

$$CSF(fx, ft) = \frac{1}{\Delta A_{UMB}(fx, ft)}$$

And analogously, in channels T and D:

$$CSF(fx, ft) = \frac{1}{\Delta T_{UMB}(fx, ft)}$$

$$CSF(fx, ft) = \frac{1}{\Delta D_{UMB}(fx, ft)}$$

About spatial and temporal characterization of stimuli

The patterns generated by default through the device are space and time sinusoidal grids, with the vertical bands by default, and with a two dimensional gaussian envelope. The spatial-temporal profile of this pattern, referred to as Gabor stimulus, can be described as follows:

$$\begin{pmatrix} \Delta A(x,t) \\ \Delta T(x,t) \\ \Delta D(x,t) \end{pmatrix} = \begin{pmatrix} \Delta A_0 \\ \Delta T_0 \\ \Delta D_0 \end{pmatrix} sen\, 2\pi f_e x\, sen\, 2\pi f_t t \cdot exp\left\{ -\frac{(x^2 + y^2)}{\sigma^2} \right\} rect\left( \frac{x}{a}, \frac{y}{a} \right) g(t)$$

where:

$$g(t) = \begin{cases} e^{-\frac{(t-t_1)^2}{2\sigma^2}} & if \quad 0 \le t \le t_1 \ ms \\ 1 & if \quad t_1 < t \le t_2 \ ms \\ e^{-\frac{(t-t_2)^2}{2\sigma^2}} & if \quad t_2 < t \le t_e \ ms \end{cases}$$

The number of pixels per cycle necessary to generate the frequency that is requested is rounded off to the closer integer value by the developed program and therefore not all the generated frequencies are going to correspond with the desired frequency. Therefore, in order the generated frequency corresponds with the desired one, only frequencies which can be generated with a whole number of pixels per cycle will be requested. This condition involves that the requested frequencies must be sampling frequency divisors. On the other hand, in order the generated grid profile is as sinusoidal as possible, those which are particularly even divisors of the sampling frequency will be required among the frequencies fulfilling the above requirement, which is, by default, 16 cycles by degree at the observation distance.

The conditions to be fulfilled by temporal frequencies are the same as for spatial frequencies. The frequency of the temporal sampling is, by default, 72 Hz.

Spatial and temporal frequencies generated by the device are 0, 0,5, 2, 4, 8 and 16 cycles/degree in the spatial domain, and 0, 2, 6, 12, 18 (in T and D), 24 (in A) cycles/second in the temporal domain. Note that 24 Hz is not an even divisor of the sampling frequency; it was however chosen among the discrete set of reproducible frequencies for gathering information in the high frequencies area in the achromatic channel.

Gaussians that modulate the grids were chosen with standard deviations, $\alpha$, equal to 1/6 of the image size, with this subtending 5° by default. Considering that a gaussian tends to zero $3\alpha$ away from its center, this choice ensures that no gabor cutoffs essentially exist in any direction of the space. Grids and gabors with certain spatial frequency in the cardinal directions of the color space are shown in Figure 2.

Function g(t) is a square temporal envelope with a length $t_e$, 1 second by default, (if the push button has not been pressed before), with gaussian smoothings at the beginning and the end of the presentation. Said smoothings, centered at $t_1$ and $t_2$, have a length equal to 10% of the total length $t_e$. A random length pause is introduced between presentations.

About the tested sectors of the retina

The device allows the sensitivity of the retina to be evaluated in a field of sight of up to $\pm$ 40° in vertical and $\pm$ 60° in horizontal. In order to explore different areas of the field of sight the position of the fixation point can be modified. Particularly, with the point of fixation being at the center of the screen (position by default) the examined field of sight is $\pm$20° in vertical and $\pm$ 30° in horizontal, segmenting it from 4 rows and 6 columns (24 sectors) to 8 rows and 12 columns (96 sectors), in any case removing the 4 sectors in the corners and with a specific sector for the fovea, that is, from 21 sectors to 93 sectors in total.

About the experimental determination of the blind spot

The measurement session begins with the determination the patient's blind spot position and size, according to the algorithm created for performing this task. Once the fixation stimulus suitable for the patient has been chosen, on an achromatic background with luminance being half of the monitor maximum luminance, an achromatic square stimulus test of the maximum luminance allowed by the device is presented, subtending 1° and with a 1 second of total length. Test position is caused to be varied by scanning the field of sight in horizontal direction first and then in vertical position, in straight lines passing through the center of an average standard individual's blind spot. The patient, looking at the fixation point, presses the push button as long as test stimulus is visible. The blind spot size is calculated from the size of the space area where the patient does not respond to the test. The center of said area will be used in the step of threshold measurement for presenting the stimuli checking suspected fixation losses.

About the measurement method

The spatial frequency, the temporal frequency and the direction of modulation define a measurement session. Once position and size of the patient's blind spot has been determined, the measurement session as such begins with the occurrence of the fixation stimulus and with the display screen being turned on with the average stimulus (remember that it is the screen white by default). The patient will be adapted during at least 30 seconds to said average stimulus. Once this period of time has been completed, the fixation stimulus disappears and the first test appears. The test position will randomly vary on the screen during session, and the patient will press the push button if any spatial-temporal variation is detected with respect to the average at any position of his/her field of sight. During session, the whole screen is still on with the average stimulus. In order a detection is counted the stroke has to be done between $t_1$ seconds from the appearance of the stimulus and disappearance thereof. The algorithm designed for this purpose will decide, depending upon whether a stimulus is detected or not, if next time that a stimulus is going to be presented in that position it has to do it with a greater or smaller amplitude. In this way, all campimetry thresholds are determined in a single session.
The observation distance is, by defect, 25 cm. Measurements must be monocular and with a pertinent refracting compensation. The patient must be able to fix the glance at the observation distance during an extended period of time without feeling discomfort, and therefore it is advisable to minimize the effort of accommodation. Measurements must be always made in the dark.

About threshold search strategy

Session begins presenting a stimulus with the maximum possible value of the amplitude, $\Delta_{MAX}$. If stimulus is detected by the patient, the amplitude is divided by 2 and it is continued in this way until no detection occurs for certain value of amplitude, $\Delta_1$. A change in the direction of response is referred to as reversal. For the following presentation the amplitude will be multiplied by $\sqrt{2}$ and it will be continued in this way until detection again occurs. After this second reversal, amplitude will be divided by $\sqrt[4]{2}$, and so on. Therefore, two consecutive amplitude values are related by the expression:

$$log_2(\Delta_i) = log_2(\Delta_{i-1}) + \frac{(-1)^{n+1}}{2^n}$$

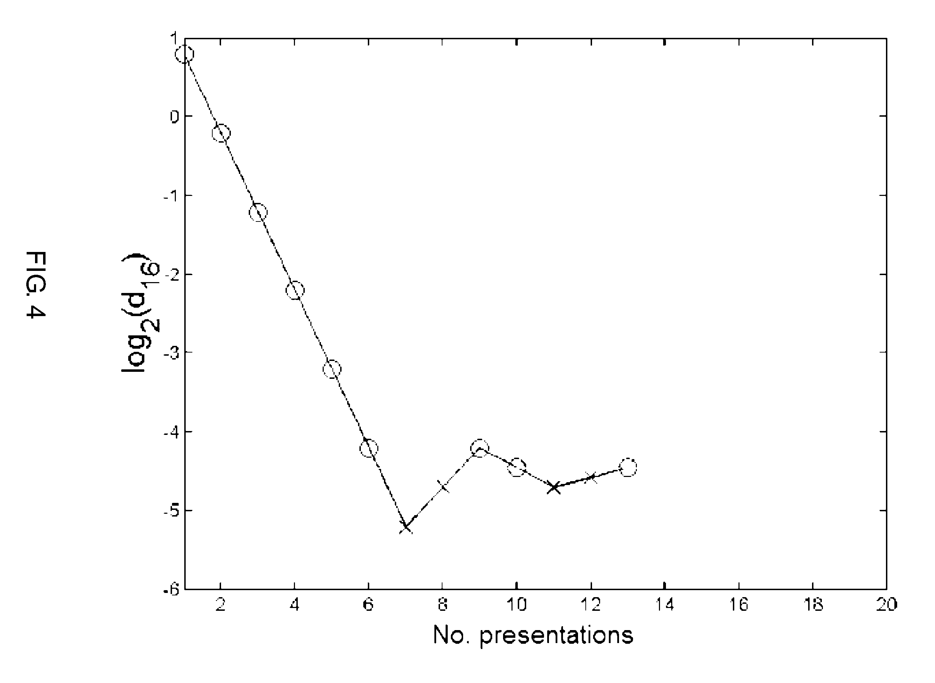

where n is the number of reversals until position i. The process is completed when a certain number of presentations is reached, by default 18 presentations (20 in the cardinal direction of A) or when 4 reversals have occurred. The last detected value of the amplitude is then allocated to threshold. Figure 4 shows an example showing this method. The method is also interrupted if 5 consecutive presentations with maximum amplitude in certain sector has not been detected. In this case and in any other case in which the threshold cannot be measured, the label *not a number* is allocated thereto.

About the control stimuli

Throughout the execution of the experiment several stimuli are semi-randomly delivered for estimating the reliability of the observer measurements:

- <u>False positive controls</u>: they are null amplitude presentations for checking a possible indiscriminate activation of the push button. In order a measurement session is considered valid the false positive rate must be lower than 33%.
- <u>False negative controls</u>: they are maximum amplitude presentations appearing near the fovea for checking a possible observer's attention deficit. These controls are similar to Gabors of the measurement that is being carried out, unless their $f_x$ and $f_t$ cannot take very high values. In order a measurement session is considered valid the false negative rate must be lower than 33%.
- <u>Fixation loss controls</u>: they are presentations in the observer's blind spot. They are 1.5° sized squares with no Gaussian smoothing. They have the chromatic and spatial modulation of the measurement that is being carried out and they do not have temporal blinking. In order a measurement session is considered valid the fixation loss rate must be lower than 20%.

In order all the controls of one class are not concentrated in time, it is proceeded as follows. $\{E_1,. E_2,..., E_i,... E_{Nmáx}\}$ is the stimuli series that could be potentially presented to the observer. A certain stimulus $E_i$ can be a threshold measurement stimulus or a control stimulus. $N_{máx}$ is the sum of the maximum number of presentations allowed for measuring a threshold in the total of the retina sectors to be explored in campimetry and the maximum number of control stimuli of all the types. The presentations corresponding to, for example, false negative controls are determined by dividing the sequence of possible stimuli in as many intervals as controls of this type is desired to present, and one of these controls being randomly locating at each interval. For example, if the potential stimuli sequence is $\{E_1, E_2,..., E_i,... E_{12}\}$ and 3 false positive controls is desired to present, 1 is randomly positioned in each of the three intervals bounded by $\{E_{1+4\ (k-1)}\ E_{4+4\ (k-1)}\}$, with k= 1,2,3, and the two remaining stimuli of each interval would be potential stimuli for measuring the threshold or potential stimuli of false negative or fixation loss. Once the false positives have been arranged in the sequence, it is proceed in the same way with the rest of controls.

<u>About standard observers</u>

The instrument is provided with a database that includes the results of the standard observers (observer with normal vision and with no pathologies), corresponding to campimetries in the cardinal directions of the color space, for each of the 25 combinations of spatial and temporal frequencies generated by the device by default.
These measurements will be used for making the corresponding diagnoses.

<u>About result presentation</u>

Patient's results can be shown in the following formats:

a) For each modulation direction:

- Threshold amplitude matrix and error matrix for each of the spatial frequency and the temporal frequency combinations being examined.
- Absolute sensitivity campimetry in gray levels for each of the spatial frequency and the temporal frequency combinations examined.
- Diagnoses map in color code according to criteria that will be described in the following section.

b) For each sector of the retina and modulation direction and whenever sufficient measurements are available:

- Spatial-temporal detection surface of the channel for a particular retina sector.
- Spatial contrast sensitivity functions (CSFs) at a constant temporal frequency.
- Temporal contrast sensitivity functions (CSFs) at a constant spatial frequency.

<u>About diagnosis criteria</u>

With the purpose of determining whether a patient's response is within the normality interval, the probability p that normal measurements (according to standard observers) exist is determined over the measurement carried out by the patient. Thus, measurements carried out are codified as follows: in green when said probability p is greater than 5% ($p \geq 0,05$), yellowish green when ranging from 5% to 2% and ($0,02 \leq p < 0,05$), yellow when ranging from 2% to 1% ($0,01 \leq p < 0,02$), orange when ranging from 1% to 0,5% ($0,005 \leq p < 0,01$), red when being lower than 0,5% ($p < 0,005$). Remember that is not always possible to carry out measurements of the threshold. As noted above, when measurement has not been able to be carried out a *not a number* label is allocated thereto. When measurement is *not a number* (maximum contrast is not seen by the patient) it is codified in blue when probability that a normal individual is also labeled for that measurement with *not a number* (that is, he/she does not see the maximum contrast either) is greater than or equal to 50%. When this percentage is ranging from 25% to 50% it is codified in violet. Finally, when less than 25% of normal individuals do not see the stimulus either, it is codified with a purple color.

About other embodiments

The invention has been described referring to one embodiment, but one skilled in the art will be able to introduce variations and will be able to replace some elements by others technically equivalent, which also will be included within the scope of protection defined by the appended claims.

For example, the device could be divided into several non-integrated elements, or elements of the computer system that have been presented separately could be integrated.

**Claims**

1. Device to determine the contrast sensitivity of an individual's visual system by means of a series of tests in a measurement session, comprising means for producing real time digital images, corresponding to modulated visual stimuli, and display means (D) for presenting said images to an individual's eye, **characterized in that** said stimuli are modulated around an average stimulus in any direction of the color space and can be configured in their spatial and temporal characteristics for each measurement session.

2. Device as claimed in claim 1, wherein said stimuli are chosen in directions of the color space in which only one of the mechanisms A, T or D is able to respond to the stimulus.

3. Device as claimed in claim 1 or claim 2, wherein said average stimulus corresponds to the white color of the display means (D).

4. Device as claimed in any of the preceding claims, wherein these stimuli are modulated sinusoidally in time.

5. Device as claimed in any of the preceding claims, wherein the stimuli respond to a pattern with spatial and temporal modulation that comprises a sinusoidal grid.

6. Device as claimed in claim 5, wherein said sinusoidal grid is modulated by a two dimensional gaussian envelope.

7. Device as claimed in any of claims 5 or 6, wherein the spatial frequency of said pattern is selected from 0 to 20 cycles/degree.

8. Device as claimed in any of claims 5 to 7, wherein spatial modulation of the pattern takes the horizontal direction.

9. Device as claimed in any of claims 4 to 8, wherein temporal frequency of said pattern is selected from 0 to 30 cycles/ second.

10. Device as claimed in any of the preceding claims, wherein each stimulus length is selected from 0 to 5 seconds.

11. Device as claimed in any of the preceding claims, wherein the colors included in the modulated stimuli in a particular direction of the color space is extracted from a 256 color palette generated for said direction.

12. Device as claimed in any of the preceding claims, wherein it comprises a (CCD) camera for recording the eye position during a test of the measurement session.

13. Device as claimed in any of the preceding claims, wherein it comprises means for the individual to hold his/her head and gaze as steady as possible in each test of the measurement session.

14. Device as claimed in claim 13, wherein said means comprise a chin rest with occluder and support for lenses.

15. Device as claimed in any of the preceding claims, wherein it examines the individual's field of sight up to 20° in vertical and 30° in horizontal around a fixation point.

16. Device as claimed in claim 15, wherein it allows the fixation point to be moved up to 20° in vertical and 30° in horizontal.

17. Device as claimed in any of the preceding claims, wherein, for its examination, the individual's field of sight is divided into sectors, establishing a specific sector for the fovea.

18. Device as claimed in any of the preceding claims, wherein said image producing means and said displaying means (D) form part of a computer system that also comprises a central processing unit (UCP) and a memory (M).

19. Device as claimed in claim 18, wherein said memory (M) comprises: a measurement session configuration and control routine; a repository of results of the measurement session; a repository of results obtained for individuals with no visual pathologies, referred to as standard observers; a visual pathology diagnosis routine from the results of the measurement session and said standard observers; a displaying routine of said results and said diagnosis

20. Device as claimed in claim 19, wherein said routine of measurement session configuration and control comprises: a stimuli configuration subroutine; a measurement method configuration subroutine; a spatial and temporal randomization subroutine of stimuli presentation; a stimuli amplitude selection subroutine; a real time recording subroutine of the results of the measurement session.

21. Device as claimed in any of the preceding claims, wherein it comprises means (P) for indicating detection, if this is the case, on the part of the individual of a spatial-temporal change with respect to the average stimulus.

22. Method to determine contrast sensitivity of an individual's visual system by means of a series of tests in a measurement session, **characterized in that** it comprises the use of a device as claimed in any of the claims 1 to 21.

23. Method as claimed in claim 22, wherein during the measurement session the individual is able to indicate detection, if this is the case, of a spatial-temporal change with respect to the average stimulus.

24. Method as claimed in claim 23, wherein in order a detection is counted the individual has to indicate it after a period of time from the appearance of the stimulus but before its disappearance.

25. Method as claimed in any of the claims 22 to 24, wherein during the measurement session the position in which each test stimulus is presented to the eye is randomly varied and the pause between one stimulus and the following one is also randomly varied.

26. Method as claimed in any of the claims 22 to 25, wherein it comprises a step of determining the individual's blind spot position and size in which a high luminance, substantially point shaped, short visual stimulus is presented to the individual's eye successively moving in horizontal and vertical directions, the individual being able to press a push button when the stimulus is visible to him/her, so that the blind spot position and size are calculated from the area in which the individual does not respond to the stimulus.

27. Method as claimed in any of the claims 22 to 26, wherein the measurement session comprises the steps of: presenting a stimulus with the maximum possible value of the amplitude; repeating stimulus presentation by dividing the amplitude successively by 2 as long as a stimulus is detected by the individual, that is, until reversal occurs; repeating stimulus presentation multiplying successively by $2^{1/2}$ the amplitude from non detection until the stimulus has been detected again by the individual, that is, until a new reversal occurs; carrying out both preceding steps but applying a division or multiplication factor of $_2 2^{-n}$, respectively, with n being the reversal number; finishing the session after 4 reversals or 20 presentations; allocating the last amplitude value detected to the threshold; and determining contrast sensitivity as the inverse of said threshold.

28. Method as claimed in any of the claims 22 to 27, wherein the measurement session comprises the semi-random presentation of control stimuli of false positives, false negatives and fixation loss.

29. Method according to anyone of claims 22 to 28, wherein the method comprises the use of an algorithm of selecting the parameters of each test based upon the results of measurement session previous tests.

FIG. 1

FIG. 2

FIG. 3

FIG. 4

## INTERNATIONAL SEARCH REPORT

| | |
|---|---|
| International application No. | |
| PCT/ ES 2005/070109 | |

**A. CLASSIFICATION OF SUBJECT MATTER**

**See additional sheet**

According to International Patent Classification (IPC) or to both national classification and IPC

**B. FIELDS SEARCHED**

Minimum documentation searched (classification system followed by classification symbols)

A61B3

Documentation searched other than minimum documentation to the extent that such documents are included in the fields searched

Electronic data base consulted during the international search (name of data base and, where practicable, search terms used)

CIBEPAT,EPODOC,WPI,PAJ,INSPEC,MEDLINE,NPL

**C. DOCUMENTS CONSIDERED TO BE RELEVANT**

| Category* | Citation of document, with indication, where appropriate, of the relevant passages | Relevant to claim No. |
|---|---|---|
| A | US 20030174284 A (Stewart) 18.09.2003 <br> **The whole document** | 1 - 29 |
| A | US 20030081176 A (Stewart) 01.05.2003 <br> **The whole document** | 1 - 29 |
| A | WO 0069327 A (VISIONRX.COM) 23.11.2000 <br> **The whole document** | 1 - 29 |
| A | US 5539482 A (James et al.) 23.07.1996 <br> **The whole document** | 1 - 29 |
| A | WO 9529627 A (The Australian National University) 09.11.1995 <br> **The whole document** | 1 - 29 |
| A | US 6623118 B (De La Rosa) 23.09.2003 <br> **The whole document** | 1, 4, 5 |

☐ Further documents are listed in the continuation of Box C.    ☒ See patent family annex.

| | |
|---|---|
| *   Special categories of cited documents: <br> "A"   document defining the general state of the art which is not considered to be of particular relevance <br> "E"   earlier document but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search <br> **16 NOV 2005 (16.11.05)** | Date of mailing of the international search report <br> **22 NOV 2005 (22.11.05)** |
|---|---|
| Name and mailing address of the ISA/ <br><br> **S.P.T.O.** <br> Facsimile No. | Authorized officer <br><br><br> Telephone No. |

Form PCT/ISA/210 (second sheet) (July 1992)

## INTERNATIONAL SEARCH REPORT
Information on patent family members

International Application No

PCT/ ES 2005/070109

| Patent document cited in search report | Publication date | Patent familiy member(s) | Publication date |
|---|---|---|---|
| US 2003174284 A | 18.09.2003 | US 6755529 B | 29.06.2004 |
| US 20030081176 A | 01.05.2003 | US 6742894 B | 01.06.2004 |
| WO 0069327 A | 23.11.2001 | MX 2001011658 A | 01.09.2003 |
| | | US 6068377 A | 30.05.2000 |
| | | AU 200048472 A | 05.12.2000 |
| | | EP 1185196 A | 13.03.2002 |
| | | BR 200010585 A | 28.05.2002 |
| | | KR 2002013547 A | 20.02.2002 |
| | | JP 2002543906T | 24.12.2002 |
| | | CN 1377243 A | 24.12.2002 |
| US 5539482 A | 23.07.1996 | AU 3386493 A | 02.09.1993 |
| WO 9529627 A | 09.11.1995 | US 5912723 A | 15.06.1999 |
| | | AU 2340495 A | 29.11.1995 |
| | | EP 0758862 A | 26.02.1997 |
| US 6623118 B | 23.09.2003 | DE 10115508 A | 25.10.2001 |
| | | JP 2001299700 A | 30.10.2001 |
| | | US 2002047996 A | 25.04.2002 |

Form PCT/ISA/210 (patent family annex) (July 1992)

## INTERNATIONAL SEARCH REPORT
Information on patent family members

| International Application No |
| --- |
| PCT/ ES 2005/070109 |

| Patent document cited in search report | Publication date | Patent familiy member(s) | Publication date |
| --- | --- | --- | --- |

**IPC 8**

*A61B3/02 (2006.01)*
*A61B3/06 (2006.01)*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5065767 A
- US 6068377 A
- US 6227668 B
- WO 9529627 A